# EUROPÄISCHE PATENTSCHRIFT

(11) **EP 1 448 785 B1**
(45) Veröffentlichungstag und Bekanntmachung des Hinweises auf die Patenterteilung: **03.10.2007**
(21) Anmeldenummer: 02803034.4
(22) Anmeldetag: 15.11.2002
(51) Int. Cl.: C12P 7/62, C12P 19/00, C12P 19/02, C12P 19/12, C12P 19/44

(54) **ENZYMATISCHE SYNTHESE VON ZUCKERACRYLATEN**
ENZYMATIC SYNTHESIS OF SUGAR ACRYLATES
SYNTHESE ENZYMATIQUE D'ACRYLATES DE SUCRE

(30) Priorität: 16.11.2001 DE 10156352
(43) Veröffentlichungstag der Anmeldung: 25.08.2004
(73) Patentinhaber: BASF Aktiengesellschaft, 67056 Ludwigshafen (DE)
(72) Erfinder: BOECKH, Dieter, 67117 Limburgerhof (DE); HAUER, Bernhard, 67136 Fussgönheim (DE); HÄRING, Dietmar, 67059 Ludwigshafen (DE)
(74) Vertreter: Reitstötter - Kinzebach
(86) Internationale Anmeldenummer: PCT/EP2002/012840
(87) Internationale Veröffentlichungsnummer: WO 2003/042227

(56) Entgegenhaltungen:
- WO-A-94/14823
- DE GOEDE, A.T.J.W. ET AL: "Selective lipase-catalyzed 6-O-acylation of alkyl alpha-D-glucopyranosides using functionalized ethyl esters" RECUEIL DES TRAVAUX CHIMIQUES DES PAYS-BAS, Bd. 112, November 1993 (1993-11), Seiten 567-572, XP008022342 in der Anmeldung erwähnt
- PARK HYUN GYU ET AL: "Enzymatic regioselective synthesis of sucrose acrylate esters." BIOTECHNOLOGY LETTERS., Bd. 22, Nr. 1, Januar 2000 (2000-01), Seiten 39-42, XP002255426 ISSN: 0141-5492
- MARTIN BD ET AL: "Biocatalytic synthesis of sugar-containing poly(acrylate)-based hydrogels" MACROMOLECULES, Bd. 25, Nr. 26, 21. Dezember 1992 (1992-12-21), Seiten 7081-7085, XP002255427
- DE GOEDE, A.T.J.W., ET AL.: "Selective lipase-catalysed esterification of alkyl glycosides" BIOCATALYSIS, Bd. 9, 1994, Seiten 145-155, XP008022345 in der Anmeldung erwähnt
- KUMAR RAJESH ET AL: "Biocatalytic route to well-defined macromers built around a sugar core." JOURNAL OF THE AMERICAN CHEMICAL SOCIETY, Bd. 124, Nr. 9, 6. März 2002 (2002-03-06), Seiten 1850-1851, XP002255428 March 6, 2002 ISSN: 0002-7863 -& KUMAR ET AL: "Supporting Information: Biocatalytic route to well-defined macromers built around a sugar core." JOURNAL OF THE AMERICAN CHEMICAL SOCIETY (SUPPORTING INFORMATION), 2002, Seiten S1-S7, XP002255429
- LI YANZI ET AL: "Scale-up of pseudo solid-phase enzymatic synthesis of alpha-methyl glucoside acrylate." BIOTECHNOLOGY AND BIOENGINEERING, Bd. 79, Nr. 1, 5. Juli 2002 (2002-07-05), Seiten 15-22, XP002255430 ISSN: 0006-3592
- ISAO IKEDA ET AL: "LIPASE-CATALYZED ACYLATION OF SUGARS SOLUBILIZED IN HYDROPHOBIC SOLVENTS BY COMPLEXATION" BIOTECHNOLOGY AND BIOENGINEERING, INTERSCIENCE PUBLISHERS, LONDON, GB, Bd. 42, Nr. 6, 1993, Seiten 788-791, XP002025123 ISSN: 0006-3592
- CHEN X. ET AL: "Enzymatic and chemoenzymatic approaches to synthesis of sugar-based polymer and hydrogels" CARBOHYDRATE POLYMERS, APPLIED SCIENCE PUBLISHERS, LTD. BARKING, GB, Bd. 28, Nr. 1, 1995, Seiten 15-21, XP004034426 ISSN: 0144-8617

## Beschreibung

Die Erfindung betrifft ein Verfahren zur enzymatischen Synthese von Zuckeracrylaten sowie ein Verfahren zur Herstellung polymerer Zuckeracrylate, die nach diesem Verfahren erhältlichen Polymere und deren Verwendung zur Herstellung von beispielsweise Kosmetika, Pharmazeutika, Waschmittel, Verdickem, Schutzkolloiden, Superabsorbern und Textilschlichten.

### Stand der Technik:

Die Zuckeracrylate sind auf verschiedenen Wegen zugänglich. Die gezielte chemische Synthese von Zuckeracrylaten ist wegen der hohen Funktionalität der Zuckermoleküle schwierig. Mit Hilfe von Schutzgruppen sind aufwendige und teure mehrstufige Synthesen von Monoacrylsäureestem verschiedener Zucker beschrieben worden. Die direkte Ver- oder Umesterung von Acrylsäure oder Acrylsäureestern mit Zuckern führt nur bei niederen Umsätzen (<20 %) zur Monoacrylsäureestern des Zuckers. Bei höheren Umsätzen kommt es zur unselektiven Bildung von Mehrfachestern. Diese können nur durch aufwendige chromatographische Verfahren aufgetrennt werden. Die Verwendung aktivierter Acrylsäurederivate, wie z.B. Acrylsäurechlorid, kann zwar die Reaktionszeiten verkürzen, führt aber ebenfalls zur unselektiven Veresterung der Zucker.

Bei der biokatalytischen Synthese beschreitet man bisher in wesentlichen zwei verschiedene Wege. Der erste Herstellungsweg verläuft über die Verwendung aktivierter (Meth)Acrylsäurederivate. Insbesondere wurden Synthesen mit Vinyl(meth)acrylat (z.B. Chen et al., Macromol. Chem. Phys. 1994, 195, 3567-3578; Chan und Ganem, Biocatalysis 1993, 8, 163-169; Park und Chang, Biotechnol. Lett. 2000, 22, 39-42; Ivanova et al., Prikladnaa biohimia i mikrobiologia 1997, 33, 269-274);

Butandiolmonooximester von (Meth)acrylsäure (Panarin et al., Vysokomolekulyamye Soedineniya Seriya A & Seriya B 1998, 40, 15-23); oder Trifluoroethyl(meth)acrylat (Potier et al., Tetrahedron Lett. 2000, 41, 3597-3600) beschrieben. Solche aktivierten Acrylsäurederivate sind wegen ihrer hohen Herstellkosten für eine wirtschafltiche Synthese von Zuckeracrylaten nicht von Interesse.

Der zweite Weg der biokatalytischen Herstellung von Zuckeracrylaten verläuft über die enzymatische Umesterung von Alkylglucosiden mit Alkylacrylaten (Goede et al., Biocatalysis 1994, 9, 145-155; Goede et al., Recl. Trav. Chim. Pays-Bas 1993, 112, 567-572; Goede et al., Heterogeneous Catalalysis and Fine Chemicals III (Hrsg. Guisnet et al.) Elsevier Science Publishers 1993, S. 513-520).

Die Nachteile der darin beschriebenen Synthesen sind zu sehen in:a) den großen Reaktionsvolumina wegen der Verwendung niederer Zuckerkonzentration (0,06-0,1 mol/l); b) dem hoher molerer Überschuß an Acrylat (etwa 44-134-facher Überschuß); c) dem hoher Volumenanteil an organischem Lösungsmittel (Zugabe von etwa 5 ml tert.-Butanol pro mmol Zucker).

In der JP-A-11028096 wird ein spezielles Rührsystem für die Umsetzung von Zuckern mit großen Viskositätsunterschieden beschrieben. Mit Hilfe von sogenannten "gate blades" gelang die enzymatische Umsetzung von Alkylglucosiden mit Alkylacrylaten. In einem Vergleichsbeispiel (Umsetzung Butylglucosid mit Methylacrylat) mit einem anderen Rührer wurde kein Umsatz erhalten. In sämtlichen Ausführungsbeispielen erfolgte die Umsetzung ohne Zugabe eines organischen Lösungsmittels.

Als Nachteile dieses Verfahrens sind anzusehen:a) die Verwendung eines speziellen Rührwerk als Voraussetzung für die Reaktion; b) der hohe molare Überschuß von Methylacrylat über Zucker (42 - 52-fach), sowie c) die hohen Reaktionstemperaturen (bevorzugt 50-80 °C), welche eine schnelle Denaturierung von Enzymen und erhöhte Polymerisationsneigung von Acrylaten zur Folgehaben, und den Zusatz von Stabilisatoren erfordern; und d) die Verwendung große Enzymmengen (mind. 10 Gew.-%; in Ausführungsbeispielen 20-100 Gew.-%).

Die US-A-5,240,835 beschreibt die enzymatische Synthese von ungesättigten polymerisierbaren Monomeren aus einem ungesättigten Ester und einer organischen Verbindung mit einer primären oder sekundären Hydroxylgruppe unter Verwendung eines aus Corynebacterium oxydans abgeleiteten Biokatalysators. Die Umsetzung erfolgt in wässriger Umgebung ohne Gegenwart eines organischen Lösungsmittels. Der Ester wird vorzugsweise in hohem molaren Überschuß eingesetzt (etwa 50 bis 120-fach). Die erfolgreiche Umsetzung von Zuckern ist durch kein einziges Ausführungsbeispiel belegt. Nachteilig ist hierbei anzusehen: a) die Notwendigkeit der Isolierung eines speziellen Enzyms aus C. oxydans; und b) der hohe molare Überschuß von Ester zu Alkohol.

### Kurze Beschreibung der Erfindung

Aufgabe der Erfindung war es, ein Verfahren zur Herstellung von Zuckeracrylaten zu entwickeln das die oben beschriebenen Nachteile des Standes der Technik wenigstens teilweise vermeidet. Die Synthese sollte insbesondere bei guter Ausbeute an gewünschen Zucker-Monoacrylat, selektiv, d.h. ohne Bildung von Mehrfachestern in kostengünstiger Weise durchführbar sein.

Obige Aufgabe konnte überraschenderweise durch gezielte Wahl der Verfahrensbedingungen, insbesondere durch Arbeiten in einem organischen Lösungsmittel-Milieu bei relativ geringem absolutem Anteil an organischem Lösungsmittel (bezogen auf die eingesetzte Zuckermenge) gelöst werden.

### Detaillierte Beschreibung der Erfindung

Ein erster Gegenstand der Erfindung betrifft ein Verfahren zur enzymatischen Synthese von Zuckeracrylaten, wobei man eine Zuckerverbindung in Gegenwart eines Acrylatgruppen übertragenden Enzyms in einem ein organisches Lösungsmittel umfassendes, flüssiges Reaktionsmedium mit einer Acrylsäureverbindung oder einem Alkylester davon gemäß der Definition in Anspruch 1 umsetzt, wobei das organische Lösungsmittel in einem Anteil von weniger als etwa 4,8 ml pro mmol Zuckerverbindung enthalten ist; und das gebildete Zuckeracrylat nach Beendigung der Reaktion gegebenenfalls aus dem Reaktionsgemisch isoliert.

Als organische Lösungsmittel finden vorzugsweise solche Verwendung, die ausgewählt sind unter Monoolen, wie C₃-C₆-Alkanolen, insbesondere tert.- Butanol und tert.-Amylalkohol, Pyridin, Poly-C₁-C₄-alkylenglykoldi-C₁-C₄-alkylethern, insbesondere Polyethylenglycoldi-C₁-C₄-alkylether, wie z.B. Dimethoxyeethan, Diethylenglycoldimethylether, Polyethylenglycoldimethylether 500, C₁-C₄-Alkylencarbonaten, insbesondere Propylencarbonat, C₃-C₆-Alkylessigsäureestem, insbesondere tert.-Butyl-essigsäureester, Aceton, 1,4-Dioxan, 1,3-Dioxolan, THF, Dimethoxymethan, Dimethoxyethan, sowie deren ein- oder mehrphasigen Mischungen.

Das organische Lösungsmittel wird vorzugsweise in einem Anteil von 0,01 bis 4, bevorzugt 0,1 bis 3 ml/mmol Zuckerverbindung eingesetzt wird. Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige verdünnte (z.B. 10 bis 100mM) Puffer, beispielsweise mit einem pH im Bereich von etwa 6 bis 8, wie z.B. Kaliumphosphat- oder TRIS-HCI-Puffer.

Die Substrate liegen entweder gelöst, als Feststoffe suspendiert oder in Emulsion im Reaktionsmedium vor. Vorzugsweise liegt die anfängliche Zuckerkonzentration im Bereich von etwa 0,1 bis 20 Mol/l, insbesondere bei 0,15 bis 10 Mol/l oder 0,2 bis 5 mol/l liegt.

Die erfindungsgemäß eingesetzten Zuckerverbindungen sind offenkettige und cyclische Mono-, Oligo- und Polysaccharide, sowie oxidierte, reduzierte, alkylierte, veresterte, aminierte Zucker aus natürlichen und synthetischen Quellen. Insbesondere sind die Zuckerverbindungen ausgewählt unter Mono- und Oligosacchariden und den veresterbaren Derivaten davon in optisch reiner Form oder als Stereoisomerengemisch. Versesterbare Monosaccharide sind ausgewählt unter Aldosen und Ketosen, insbesondere Aldo- und Keto- Pentosen und -Hexosen und den veresterbaren Derivaten davon, insbesondere C₁-C₃₀-Alkyl-Glykosiden. Bevorzugte Oligosaccharide sind ausgewählt unter Di- und Trisacchariden und den veresterbaren Derivaten davon. Weitere mögliche Zucker sind C₁-C₃₀-Alkyl-Glykoside mit einer oder mehreren funktionellen Gruppen in der Alkylkette, sowie Glycoside, welche Polyalkylenglykol-Reste, wie z.B. Polyethylenglykol- oder Polypropylenglykol-Reste, tragen. Nichtlimitierende Beispiele für geeignete funktionelle Gruppen sind O-, S- oder N-haltige Gruppen, wie HO-, HS-, Amino-, Carboxyl- oder Carbonylgruppen sowie Ether- und Thioether-Brücken.

Bevorzugt werden C₁-C₃₀-, wie z.B. C₁-C₆-Alkylglykoside, insbesondere Methyl-glycopyranoside, Methyl-α-D-glucopyranosid, verwendet. Das Alkylglycosid kann direkt zugesetztwerden oder in der Reaktionslösung intermediär aus dem Zucker mit dem entsprechenden Alkylalkohol säurekatalysiert (z.B. mit lonenaustauschern) hergestellt werden.

Die erfindungsgemäß eingesetzte Acrylsäureverbindung ist vorzugsweise ausgewählt unter (Meth)Acrylsäure, deren Anhydride, C₁-C₆-Alkyl-substituierter Acrylsäure, den C₁-C₆-Alkylestern davon oder Ethylenglykoldiacrylate. Erfindungsgemäße Acrylsäure-Verbindungen umfassen sowohl unsubstituierte als auch substituierte Acrylsäuren. Geeignete Substituenten sind C₁-C₆-Alkyl-Gruppen, insbesondere Methyl- oder Ethylgruppen. Bevorzugt verwendet man (Meth)Acrylsäure oder (Meth)Acrylsäurederivate.

Geeigntete (Meth)Acrylsäurederivate sind Ester mit gesättigten und ungesättigten, cyclischen oder offenkettigen C₁-C₁₀-Monoalkoholen, insbesondere Methyl-, Ethyl-, Butyl- und 2-Ethylhexyl(meth)acrylat. Die erfindungsgemäßen C₁-C₁₀-Monoalkohole umfassen bevorzugt C₁-C₆-Alkylgruppen obiger Definition oder deren längerkettigen, gegebenenfalls verzweigten, Homologen mit bis zu 10 Kohlenstoffatomen oder C₄-C₆-Cycloalkyl Gruppen, wie Cyclopropyl, Cyclopentyl oder Cyclohexyl, welche gegebenenfalls durch eine oder mehrere Alkylgruppen mit 1 bis 3 C-Atomen substituiert sein können.

Werden keine anderen Angaben gemacht so steht erfindungsgemäß C₁-C₄-Alkyl für Methyl, Ethyl, n- oder i-Propyl, n-, sec.- oder tert.-Butyl; C₃-C₆-Alkyl steht insbesondere für n- oder i-Propyl, n-, sec.- oder tert.-Butyl, n- oder tert.-Amyl, sowie geradkettiges oder verzweigtes Hexyl. C₁-C₆-Alkylgruppen umfassen die obigen Definitionen für C₁-C₄- und C₃-C₆-Alkyl. C₁-C₄-Alkylen steht vorzugsweise für Methylen, Ethylen, Propylen oder 1- oder 2-Butylen. C₁-C₃₀-Alkyl-Gruppen umfassen obige C₁-C₆-Alkylgruppen und längerkettige Reste, wie z.B. n-Heptyl, n-Octyl, n-Nonyl, n-Decyl, n-Undecyl, n-Dodecyl, n-Tridecyl, n-Tetradecyl, n-Pentadecyl und n-Hexadecyl, Octadecyl, Docosanyl sowie die ein- oder mehrfach verzweigten Analoga davon.

Die erfindungsgemäß eingesetzten Enzym sind ausgewählt unter Lipasen (E.C. 3.1.1.3) in freier oder immobilisierter Form. Besonders geeignet sind Novozyme 435 (Lipase aus *Candida antarctica* B) oder Lipase aus Aspergillus sp., Burkholderia sp., Candida sp., Pseudomonas sp., oder Schweinepankreas. Der Enzymgehalt im Reaktionsmedium im Bereich von etwa 0,1 bis 10 Gew.-%, bezogen auf die eingesetzte Zuckerverbindung liegt. Die Enzyme können in der erfindungsgemäßen Umsetzung in reiner Form oder geträgert (immobilisiert) eingesetzt werden.

Bei den erfindungsgemäßen Verfahren liegt die Reaktionstemperatur im Bereich von 0 bis etwa 70°C, bevorzugt bei 20 bis 60 °C. Die Reaktionsdauer liegt gewöhnlich im Bereich von etwa 1 bis 72 Stunden. Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion mit dem Enzympräparat versetzt. Die Temperatur wird während der Reaktion auf den gewünschten Wert eingestellt.

Das erfindungsgemäße Verfahren kann aber auch diskontinuierlich, halbkontinuierlich oder kontinuierlich in herkömmlichen Bioreaktoren durchgeführt werden. Geeignete Fahrweisen und Bioreaktoren sind dem Fachmann geläufig und z.B. beschrieben in Römpp Chemie Lexikon, 9. Auflage, Thieme Verlag, Stichwort "Bioreaktor" oder Ullmann's Encyclopedia of Industrial Chemistry, 5. Aulage, Band B4, Seiten 381ff., worauf hiermit ausdrücklich Bezug genommen wird. Der Betrieb des Reaktors und die Verfahrensführung können vom Fachmann den jeweiligen Erfordernissen der gewünschten Veresterungsreaktion angepasst werden.

Der gegebenenfalls bei der Umesterung anfallende Alkohol kann in geeigneter Weise aus dem Reaktionsgleichgewicht kontinuierlich oder schrittweise, entfernt werden. Hierzu eignen sich vorzugsweise Molekularsiebe (Porengröße z.B. im Bereich von etwa 3-10 Angström), oder eine Abtrennung durch Destillation oder mit Hilfe geeigneter semipermeabler Membranen. Die Reaktionsdauer liegt gewöhnlich im Bereich von etwa 3 bis 72 Stunden.

Nach Beendigung der Reaktion kann man das gewünschte Zuckeracrylat erforderlichenfalls, vom organischen Lösungsmittel abtrennen, z.B. chromatographisch, aufreinigen, und dann zur Herstellung der gewünschter Polymere einsetzen.

Ein weiterer Gegenstand der Erfindung betrifft ein Verfahren zur Herstellung von polymeren Zuckeracrylaten, wobei man wenigstens ein Zuckeracrylat in obiger Weise herstellt; das Zuckeracrylat aus dem Reaktionsgemisch gegebenenfalls abtrennt; und, gegebenenfalls zusammen mit weiteren Comonomeren, polymerisiert.

Geeignete weitere Comonomere sind: andere erfindungsgemäß hergestellte Zuckeracrylate des erfindungsgemäßen Typs oder polymerisierbare Nicht-Zucker-Monomere, wie (Meth)Acrylsäure, Maleinsäure, Itaconsäure, deren Alkali- oder Ammoniumsalze und deren Ester, O-Vinylester von C₁-C₂₅-Carbonsäuren, N-Vinylamide von C₁-C₂₅-Carbonsäuren, N-Vinylpyrrolidon, N-Vinylcaprolactam, N-Vinyloxazolidon, N-Vinylimidazol, (Meth)acrylamid, (Meth)acrylnitril, Ethylen, Propylen, Butylen, Butadien, Styrol. Beispiele für geeignete C₁-C₂₅-Carbonsäuren sind gesättigte Säuren , wie Ameisen-, Essig-, Propion- und n- und i-Buttersäure, n- und i-Valeriansäure, Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure.

Die Herstellung solcher Polymere erfolgt beispielsweise in Analogie zu den in "Ullmann's Enzyclopedia of Industrial Chemistry, Sixth Edition, 2000, Electronic Release, Stichwort: Polymerisation Process" allgemein beschriebenen Verfahren. Vorzugsweise erfolgt die (Co)polymerisation als radikalische Polymerisation in Form derLösungs-, Suspensions-, Fällungs-oderEmulsionspolymerisation oder durch Polymerisation in Substanz, d.h. ohne Lösemittel.

Weitere Gegenstände der Erfindung sind polymeres Zuckeracrylate, welche in obiger Weise erhältlich sind und deren Verwendung zur Herstellung von Kosmetika, Pharmazeutika, Waschmittel, Verdickern, Schutzkolloiden, Superabsorbern und Textilschlichten, Klebstoffen, Papier, Beton oder Dispersionen.

Die Erfindung wird nun anhand folgender Beispiele näher erläutert.

### Beispiel 1

Eine Mischung aus 5 mmol (0,97 g) Methyl-α-D-glucopyranosid, 50 mmol (4,3 g) Methylacrylat, 5 ml tert-Butanol, 1 g Molsieb (5 Å) und 0,1 g Novozym 435 (Lipase aus Candida antarctica B) wurde 24 h bei 40°C mit 200 Upm geschüttelt. Eine Probe wurde mit Sylilierungsreagenz (Sylon HTP) versetzt und mittels Gaschromatographie (GC) analysiert. Man fand 78 % Methyl-6-O-acryloyl-glucopyranosid, 22 % Methyl-Glucopyranosid und <1 % mehrfach veresterten Zucker.

### Beispiel 2

In einer 100 ml-Soxhlet-Apparatur wurden 20 mmol (3,9 g) Methyl-α-D-glucopyranosid, 200 mmol (17,2 g) Methylacrylat, 20 ml Aceton und 0,4 g Novozym 435 unter Rückfluß mit einem Magentrührstäbchen gerührt. Zur Entfernung des entstehenden Methanols warder Extraktionsraum mit 5 g Molsieb (5 Å) in 100 ml Aceton gefüllt. Nach 24 h wurde vom Überstand dekantiert, das Aceton im Vakuum entfernt und der ölige Rückstand (1,26 g) mittels GC analysiert. Man fand 87 % Methyl-6-O-acryloyl-Glucopyranosid, 13 % Methyl-Glucopyranosid und <1 % mehrfach veresterten Zucker.

### Beispiel 3

Es wurden 50 mmol (9,7 g) Methyl-α-D-glucopyranosid, 500 mmol (43,0 g) Methylacrylat, 50 ml Dimethoxyethan, 12,5 g Molsieb (5 Å) und 1,0 g Novozym 435 bei 60 °C gerührt. Nach 24 h wurde abfiltriert und das Filtrat im Vakuum eingeengt. Der Rückstand (8,92 g) wurde mittels GC analysiert. Man fand 84 % Methyl-6-O-acryloyl-Glucopyranosid, 16 % Methyl-Glucopyranosid und <1 % mehrfach veresterten Zucker.

### Beispiel 4

Es wurden 50 mmol (9,7 g) Methyl-α-D-glucopyranosid, 500 mmol (43,0 g) Methylacrylat, 50 ml Aceton, 12,5 g Molsieb (5 Å) und 1,0 g Novozym 435 bei 60 °C gerührt. Nach 24 h wurde abfiltriert und das Filtrat im Vakuum eingeengt. Der Rückstand (4,91 g) wurde mittels GC analysiert. Man fand 94 % Methyl-6-O-acryloyl-glucopyranosid, 6 % Methyl-Glucopyranosid und <1 % mehrfach veresterten Zucker.

### Beispiel 5

a) Es wurden 0,75 mol (145,6 g) Methyl-α-D-glucopyranosid, 7,5 mol (645,7 g) Methylacrylat, 750 ml Aceton, 187,5 g Molsieb (5 Å), 161 mg Phenothiazin und 15,0 g Novozym 435 bei 60°C gerührt. Nach 24 h wurde abfiltriert und das Filtrat im Vakuum eingeengt. Der Rückstand (91,7 g) wurde mittels GC analysiert. Man fand 92 % Methyl-6-O-acryloyl-glucopyranosid, 5 % Methyl-Glucopyranosid und 3 % eines Diacrylates von Methyl-Glucopyranosid.
b) Die Menge Molsieb wurde in obigem Ansatz auf 60 g reduziert und man erhielt 59,9 g Rückstand. Die GC-Analyse ergab eine Zusammensetzung von 92 % Methyl-6-O-acryloylglucopyranosid, 7,6 % Methyl-Glucopyranosid und 0,4 % eines Diacrylates von Methyl-Glucopyranosid.

### Beispiel 6

### a) Vergleichsversuche mit Ethylacrylat

Zur weiteren Veranschaulichung des mit der erfindungsgemäßen Lehre verbundenen überraschenden Effekts wurden folgende Vergleichsversuche durchgeführt. Dazu wurden die im folgenden beschriebenen Reaktionsansätze (A) bis (E) umgesetzt.

### Ansatz (A),Stand der Technik (vgl. Goede et al. a.a.O. 1993)

| | |
|---|---|
| 5 mmol | Methyl-α-D-glucopyranosid (0,971 g) |
| 223,7 mmol | Ethylacrylat (22,4 g; 24,3 ml) |
| 24,3 ml | tert.-Butanol |
| 97,3 mg | Novozym 435 |

1,94 g 5 Å Molsieb
72 h schütteln bei 40 °C.
Der Umsatz wurde nach Silylierung mittels GC bestimmt.

Vergleichsansätze (B) bis (E) (Bedingungen wie unter (A) sofern nichts anderes angegeben):

### Ansatz (B)

| | |
|---|---|
| 50 mmol | Ethylacrylat (5,01 g; 5,4 ml) |

### Ansatz ( C)

| | |
|---|---|
| 50 mmol | Ethylacrylat (5,01 g; 5,4 ml) |
| 43,2 ml | tert.-Butanol |

### Ansatz (D) (erfindungsgemäß)

| | |
|---|---|
| 50 mmol | Ethylacrylat (5,01 g; 5,4 ml) |
| 5 ml | tert.-Butanol |

### Ansatz (E)

| | |
|---|---|
| 50 mmol | Ethylacrylat (5,01 g; 5,4 ml) ohne tert.-Butanol |

In den Ansätzen lag Methylglucosid teilweise als Feststoff vor, d.h. die Reaktionslösung ist gesättigt mit Methylglucosid.

Die ermittelten Umsätze aus zwei identischen Ansätze und die verwendeten Reaktionsbedingungen (Molarer Überschuß an Acrylat und Lösungsmittelanteil) sind in folgender Tabelle 1 zusammengestellt:

**Tabelle 1**

| Versuch | Bedingungen | | Umsatz (%) | | |
|---|---|---|---|---|---|
| | Lösungsmittel-Anteil | Acrylat-Überschuß | 1. Ansatz | 2. Ansatz | Mittelwert |
| A | 4,8 ml/mmol | 45 × | 52,9 | 54,6 | 53,8 |
| B | 5 ml/mmol | 10 × | 32,9 | 32,5 | 32,7 |
| C | 8,6 ml/mmol | 10 × | 36,2 | 38,2 | 37,2 |
| D | 1 ml/mmol | 10 × | 47,7 | 55,5 | 51,6 |
| E | - | 10 × | 13,8 | 13,3 | 13,6 |

Nach Stand der Technik ist ein mind. 45-facher Überschuss Ethylacrylat über Methylglucosid mit viel Lösungsmittel nötig (Ansatz A; Goede et al. 1993). Wird der Überschuss an Ethylacrylat auf das 10-fache reduziert, fällt der Umsatz erwartungsgemäß. Dies gilt sowohl bei gleichbleibendem (Ansatz B), wie auch bei erhöhtem Lösungsmittel-Anteil (Ansatz C). Überraschenderweise kann der erniedrigte Umsatz durch Reduzierung des Lösungsmittel-Anteils wieder ausgeglichen werden (Ansatz D). Wird das Lösungsmittel ganz weggelassen, fällt der Umsatz dramatisch (Ansatz E).

### b) Versuche mit Methylacrylat

Gleiche Tendenzen wie mit Ethylacrylat zeigen sich auch bei Methylacrylat. Dazu wurden die folgenden Ansätze (F) bis (I) getestet.

### Ansatz (F) bis (I):

| | |
|---|---|
| 5 mmol | Methyl-α-D-glucopyranosid |
| 50 bzw. 250 mmol | Methylacrylat |
| 5 bzw. 25 ml | tert-Butanol |
| 100 mg | Novozym 435 |
| 1 g | 5 Å Molsieb |

24 h schütteln bei 40 °C. Der Umsatz wurde nach Silylierung mittels GC bestimmt. In den Ansätzen lag Methylglucosid teilweise als Feststoff vor, d.h. die Reaktionslösung ist gesättigt mit Methylglucosid.

Die ermittelten Umsätze aus zwei identischen Ansätze und die verwendeten Reaktionsbedingungen (Molarer Überschuß an Acrylat und Lösungsmittelanteil) sind in folgender Tabelle 2 zusammengestellt:

**Tabelle 2**

| Versuch | Bedingungen | | Umsatz (%) | | |
|---|---|---|---|---|---|
| | Lösungsmittel-Anteil | Acrylat-Überschuß | 1. Ansatz | 2. Ansatz | Mittelwert |
| F | 5 ml/mmol | 50 × | 53,1 | 51,2 | **52,2** |
| G | 5 ml/mmol | 10 × | 47,1 | 39,4 | **43,3** |
| H | 1 ml/mmol | 50 × | 81,9 | 77,3 | **79,6** |
| I | 1 ml/mmol | 10 × | 60,6 | 67,1 | **63,9** |

Bei einem 50-fachen Überschuss Methylacrylat über Zucker und 5 ml tert.-Butanol pro mmol Zucker erhielt man 52 % Umsatz (Ansatz F). Wird der Zuckerüberschuss verringert, fällt der Umsatz erwartungsgemäß (Ansatz G). Wird nun auch der Gehalt an Lösungsmittel auf 1 ml pro mmol reduziert, stieg der Umsatz überraschend auf 64 % an (Ansatz I, erfindungsgemäß). Durch die reduzierte Lösungsmittelmenge wurde auch bei 50-fachem Zuckerüberschuss überraschenderweise ein von 52 % (Ansatz F) auf 80 % (Ansatz H, erfindungsgemäß) erhöhter Umsatz gefunden.

## Patentansprüche

1. Verfahren zur enzymatischen Synthese von Zuckeracrylaten, wobei man eine Zuckerverbindung in Gegenwart eines Acrylatgruppen übertragenden Enzyms in einem ein organisches Lösungsmittel umfassendes, flüssiges Reaktionsmedium mit einer Acrylsäureverbindung umsetzt; das organische Lösungsmittel in einem Anteil von weniger als 4,8 ml pro mmol Zuckerverbindung enthalten ist; Acrylsäureverbindung und Zuckerverbindung in einem molaren Verhältnis von 10:1 bis 3:1 eingesetzt werden; die Acrylsäureverbindung ausgewählt ist unter Acrylsäure, C₁-C₆-Alkyl-substituierter Acrylsäure und den C₁-C₆-Alkylestern davon; das Enzym ausgewählt ist unter Lipasen gemäß E.C. 3.1.1.3 in freier oder immobilisierter Form; der Enzymgehalt im Reaktionsmedium im Bereich von 0,1 bis 10 Gew.-%, bezogen auf die eingesetzte Zuckerverbindung liegt und das gebildete Zuckeracrylat nach Beendigung der Reaktion gegebenenfalls aus dem Reaktionsgemisch isoliert.

2. Verfahren nach Anspruch 1, wobei die anfängliche Zuckerkonzentration im Bereich von 0,1 bis 20 Mol/l, insbesondere 0,15 bis 10 Mol/l liegt.

3. Verfahren nach einem der vorhergehenden Ansprüche, wobei die Zuckerverbindung ausgewählt ist unter Mono- und Oligosacchariden und den veresterbaren Derivaten davon in optisch reiner Form oder als Stereoisomerengemisch.

4. Verfahren nach Anspruch 3, wobei das Monosaccharid ausgewählt ist unter Aldosen und Ketosen, insbesondere Aldo- und Keto- Pentosen und -Hexosen und den veresterbaren Derivaten davon, insbesondere Niedrigalkyl-Glykosiden; und wobei das Oligosaccharid ausgewählt ist unter Di- und Trisacchariden und den veresterbaren Derivaten davon.

5. Verfahren nach einem der vorherigen Ansprüche, wobei das organische Lösungsmittel ausgewählt ist unter C₃-C₆-Alkanolen, insbesondere tert.- Butanol, tert.-Amylalkohol, Pyridin, Polyalkylenglykoldialkylether, Alkylencarbonat, C₃-C₆-Alkyl-, insbesondere tert.-Butyl-essigsäureester, Aceton, 1,4-Dioxan, 1,3-Dioxolan, THF, Dimethoxymethan, Dimethoxyethan, und Mischungen davon.

6. Verfahren nach einem der vorherigen Ansprüche, wobei die Reaktionstemperatur im Bereich von 0 bis 70°C liegt.

7. Verfahren nach einem der vorherigen Ansprüche, wobei das Reaktionsmedium ein- oder mehrphasig ist und worin die Reaktanden gelöst, suspendiert oder emulgiert vorliegen.

8. Verfahren nach einem der vorherigen Ansprüche, wobei man während der Veresterung gegebenenfalls anfallenden Alkohol aus dem Reaktionsgleichgewicht entfernt.

9. Verfahren zur Herstellung von polymeren Zuckeracrylaten, wobei man wenigstens ein Zuckeracrylat nach einem Verfahren gemäß einem der vorherigen Ansprüche herstellt; das Zuckeracrylat aus dem Reaktionsgemisch gegebenenfalls abtrennt; und, gegebenenfalls zusammen mit weiteren Comonomeren, polymerisiert.

10. Verfahren zur Herstellung von Kosmetika, Pharmazeutika, Waschmittel, Verdickern, Schutzkolloiden, Superabsorbern und Textilschlichten, Klebstoffen, Papier, Beton oder Dispersionen, wobei man ein polymeres Zuckeracrylat nach einem Verfahren gemäß Anspruch 9 herstellt und diesen in an sich bekannter Weise in Kosmetika, Pharmazeutika, Waschmittel, Verdickem, Schutzkolloiden, Superabsorbern und Textilschlichten, Klebstoffen, Papier, Beton oder Dispersionen einarbeitet.

## Claims

1. A process for the enzymatic synthesis of sugar acrylates which comprises reacting a sugar compound with an acrylic acid compound in a liquid reaction medium comprising an organic solvent in the presence of an acrylate-transferring enzyme; the organic solvent being comprised in an amount of less than 4.8 ml per mmol of sugar compound; acrylic acid compound and sugar compound being used in a molar ratio of 10:1 to 3:1; the acrylic acid compound being selected from the group consisting of acrylic acid, C₁-C₆-alkyl-substituted acrylic acid and the C₁-C₆ alkyl esters thereof; the enzyme being selected from the group consisting of lipases according to E.C.3.1.1.3 in free or immobilized form; the enzyme content in the reaction medium being in the range from 0.1 to 10% by weight, based on the sugar compound used and, if appropriate, the sugar acrylate formed, after completion of the reaction, being isolated from the reaction mixture.

2. The process according to claim 1, wherein the initial sugar concentration is in the range from 0.1 to 20 mol/l, in particular from 0.15 to 10 mol/l.

3. The process according to either of the preceding claims, wherein the sugar compound is selected from the group consisting of monosaccharides and oligosaccharides and the esterifiable derivatives thereof in optically pure form or as a stereoisomer mixture.

4. The process according to claim 3, wherein the monosaccharide is selected from the group consisting of aldoses and ketoses, in particular aldopentoses and ketopentoses and aldohexoses and ketohexoses and the esterifiable derivatives thereof, in particular low-alkyl glycosides, and the oligosaccharide is selected from the group consisting of disaccharides and trisaccharides and the esterifiable derivatives thereof.

5. The process according to one of the preceding claims, wherein the organic solvent is selected from the group consisting of C₃-C₆-alkanols, in particular tert-butanol, tert-amyl alcohol, pyridine, polyalkylene glycol dialkyl ether, alkylene carbonate, C₃-C₆-alkyl acetate, in particular tert-butyl acetate, acetone, 1,4-dioxane, 1,3-dioxolane, THF, dimethoxymethane, dimethoxyethane and mixtures thereof.

6. The process according to one of the preceding claims, wherein the reaction temperature is in the range from 0 to 70°C.

7. The process according to one of the preceding claims, wherein the reaction medium is single-phase or multiphase and in which the reactants are present in dissolved, suspended or emulsified form.

8. The process according to one of the preceding claims, wherein during the esterification any alcohol produced is removed from the reaction equilibrium.

9. A process for preparing polymeric sugar acrylates, which comprises preparing at least one sugar acrylate by a process according to one of the preceding claims, if appropriate separating off the sugar acrylate from the reaction mixture, and polymerizing the sugar acrylate, if appropriate, together with further comonomers.

10. A process for preparing cosmetics, drugs, laundry detergents, thickeners, protecting colloids, superabsorbents and textile sizes, glues, paper, concrete or dispersions, wherein a polymeric sugar acrylate is produced by a process according to claim 9 and it is incorporated in a manner known per se into cosmetics, drugs, laundry detergents, thickeners, protecting colloids, superabsorbents and textile sizes, glues, paper, concrete or dispersions.

## Revendications

1. Procédé de synthèse enzymatique d'acrylates de sucre, dans lequel une liaison sucre est amenée à réagir avec une liaison acide acrylique, en présence d'un enzyme transportant un groupe acrylate, dans un milieu réactionnel liquide comportant un solvant organique ; le solvant organique est contenu en une proportion inférieure à 4,8 ml par mmol de liaison sucre ; la liaison acide acrylique et la liaison sucre sont utilisées dans un rapport molaire de 10:1 à 3:1 ; la liaison acide acrylique est choisie parmi l'acide acrylique, l'acide acrylique substitué par un groupe alkyle en C₁-C₆, et ses esters alkyliques en C₁-C₆ ; l'enzyme est choisi parmi des lipases selon E.C. 3.1.1.3, sous leur forme libre ou immobilisée ; le taux d'enzyme dans le milieu réactionnel se situe dans la plage de 0,1 à 10 % en poids, par rapport à la liaison sucre utilisée et l'acrylate de sucre formé après achèvement de la réaction est éventuellement isolé du mélange réactionnel.

2. Procédé selon la revendication 1, dans lequel la concentration initiale en sucre se situe dans la plage de 0,1 à 20 mol/l, en particulier 0,15 à 10 mol/l.

3. Procédé selon l'une quelconque des revendications précédentes, dans lequel la liaison sucre est choisie parmi des monosaccharides et des oligosaccharides et leurs dérivés pouvant être estérifiés, sous une forme optiquement pure ou en tant que mélange de stéréo isomères.

4. Procédé selon la revendication 3, dans lequel le monosaccharide est choisi parmi des aldoses et des cétoses, en particulier des aldopentoses, des cétopentosès, des aldohexoses et des cétohexoses et leurs dérivés pouvant être estérifiés, en particulier des glucosides faiblement alkyles ; et dans lequel l'oligosaccharide est choisi parmi des disaccharides et des trisaccharides et leurs dérivés pouvant être estérifiés.

5. Procédé selon l'une quelconque des revendications précédentes, dans lequel le solvant organique est choisi parmi des alcanols en C₃-C₆, en particulier du tert-butanol, de l'alcool tert-amylique, de la pyridine, du dialkyléther de polyalkylèneglycol, du carbonate d'alkylène, des esters d'acide alkylacétique en C₃-C₆, en particulier de l'ester d'acide tert-butylacétique, de l'acétone, du 1,4-dioxanne, du 1,3-dioxolane, du THF, du diméthoxyméthane, du diméthoxyéthane, et leurs mélanges.

6. Procédé selon l'une quelconque des revendications précédentes, dans lequel la température réactionnelle se situe dans la plage de 0 à 70°C.

7. Procédé selon l'une quelconque des revendications précédentes, dans lequel le milieu réactionnel est monophasique ou pluriphasique, et dans lequel les réactifs sont présents en solution, en suspension ou en émulsion.

8. Procédé selon l'une quelconque des revendications précédentes, dans lequel de l'alcool éventuellement apparu pendant l'estérification est éliminé de l'équilibre réactionnel.

9. Procédé de préparation d'acrylates de sucre polymères, dans lequel au moins un acrylate de sucre est préparé selon un procédé selon l'une quelconque des revendications précédentes ; l'acrylate de sucre est éventuellement séparé du mélange réactionnel ; et est polymérisé, éventuellement en commun avec d'autres comonomères.

10. Procédé de fabrication de produits cosmétiques, produits pharmaceutiques, détergents, épaississants, colloïdes de protection, superabsorbants et enduits textiles, colles, papier, béton ou dispersions, dans lequel un acrylate de sucre polymère est préparé selon un procédé selon la revendication 9 et incorporé de manière connue en elle-même dans des produits cosmétiques, des produits pharmaceutiques, des détergents, des épaississants, des colloïdes de protection, des superabsorbants et des enduits textiles, des colles, du papier, du béton ou des dispersions.
